# EUROPEAN PATENT APPLICATION

(11) **EP 2 889 373 A1**
(43) Date of publication of application: **01.07.2015**
(21) Application number: 14196176.3
(22) Date of filing: 04.12.2014
(51) Int. Cl.: C12N 5/077

(54) **Dental differentiation product**

(30) Priority: 25.12.2013 TR 201315262
(71) Applicant: Yeditepe Universitesi, 34755 Istanbul (TR)
(72) Inventor: Sahin, Fikrettin, 34755 Istanbul (TR); Tasli, Pakize Neslihan, 34755 Istanbul (TR)
(74) Representative: Dericioglu Kurt, Ekin

(57) **Abstract**

The present invention relates to a dental differentiation product which obtains tooth cell from adult stem cells. Tooth cells are effectively obtained with adult stem cells that are used in tissue engineering. When stems cells are differentiating, they lose their own properties and acquire new properties. These properties are both at morphological level and gene expression level. The product of the present invention comprises C₁₃H₁₆N₂O) (melatonin), Ca(OH)₂ (calcium hydroxide), C₂₂H₂₉FOs (dexamethasone), C₃H₇Na₂O₆P ·5H₂O (disodium beta-glycerophosphate hydrate), NaF (sodium fluoride) and Pluronic chemicals.

## Description

### Field of the Invention

The present invention relates to a dental differentiation product which obtains tooth cell from adult stem cells.

### Background of the Invention

In addition to their potential of transforming into other cells in the early development and growing period, the stem cells also have specific properties such as self-renewal during cell division and ability to transform into cell types specific to any organ or tissue in the body when treated with special conditions and chemicals. Stem cells have two main sources, namely embryonic and adult tissues. Embryonic cells are obtained from embryonic cells of mammals. Human Embryonic Stem Cell (hESC) is obtained from the cell mass in the developing blastocyte and these cells have the potential of transforming into many cell lines (Jiang, Lv et al.). hESCs possess the properties of self-renewal and pluripotency (Thomson, Itskovitz-Eldor et al. 1998). Adult stem cells (ASC) can be obtained from various tissues in a developed organism. These adult stem cells are multipotent and the cell type into which they can transform varies according to the source from which they are obtained. ASCs have an important place in tissue engineering as they can be obtained almost from any tissue and can transform into many cell types (Cihova, Altanerova et al.). For example, stem cells obtained from bone marrow can transform into many cell types such as bone, cartilage, fat cells (Yamaza, Kentaro et al.). Recently ASCs are particularly used in bone, tooth, skin and cartilage tissue renewal studies (Zuk, Zhu et al. 2002; Djouad, Bouffi et al. 2009). The first successful ASC isolation was made by Friedenstein from bone marrow (Friedenstein, Chailakhjan et al. 1970). They implemented this isolation method by adhesion of the cells taken from bone marrow to plastic culture plates (Thomson, Bennett et al. 1993; Alhadlaq and Mao 2003).

According to International Society for Cellular Therapy, ASCs should be negative for CD14, CD 34 and CD45 (Pittenger, Mackay et al. 1999; Huss 2000; Hristov, Erl et al. 2003) which are surface proteins in the cell membrane and positive for CD105, CD73 and CD90 (Cihova, Altanerova et al.) The characterization comprising these phenotypic surface proteins is not specific only for ASCs but many different cell types posses it as well (Seshi, Kumar et al. 2000; Kinnaird, Stabile et al. 2004). Additionally, these characteristic reagents also cause morphological changes for stem cells (Huss 2000). ASC attracts the attention of tissue engineers also due to its properties of regulating the immune system. Francesco Dazzi reported that ASCs inhibit cell division of T lymphocytes and suppress immune system (Glennie, Soeiro et al. 2005). It is shown that ASCs, by suppressing division of T cells and preventing T cells from giving antigen response, enable to block the immune response that will occur at the concerned region (Bartholomew, Sturgeon et al. 2002; Krampera, Glennie et al. 2003). Furthermore, using the stem cells differing relative to the tissue, from which they are isolated, in tissue engineering and the fact that these cells suppress the immune system to a certain extent has been an advantage (De Bari, Dell'Accio et al. 2001). Since Human Tooth Germ Stem Cell (hTGSC), which includes Dental Pulp Stem Cell (DPSC) and Dental Follicle Stem Cell (DFSC), is obtained from the region of the developing tooth that includes neural crest, it possesses both stem cell and neural properties (Thesleff and Sharpe 1997). Isolation of ASC from the dental tissue is both effective and easy and does not raise ethical concerns since it is a waste material obtained during routine procedure (Yalvac, Rizvanov et al. 2009). Furthermore, it is shown that hTGSC has a protective effect on the motor and dopaminergic nerves in the spinal cord (Nosrat, Widenfalk et al. 2001; Nosrat, Smith et al. 2004). Since the hTGSCs obtained from the third molar tooth are of ectodermic, mesodermic and endodermic origin, they have a larger differentiation capacity in comparison to the other ASCs (Ikeda, Yagi et al. 2008). hTGSC has a great importance particularly in renewal of infected and damaged teeth and reestablishing the dentin-pulp structure (Yang, Walboomers et al. 2008; Zhang, Walboomers et al. 2008). The studies that were conducted suggest that the stem cells obtained from dental tissue are more effective in healing of dentin-pulp complex than the conventional methods (Gronthos, Mankani et al. 2000; Miura, Gronthos et al. 2003; Hargreaves, Geisler et al. 2008).

The recent studies and biomaterials and chemicals that are used facilitate development of tissue engineering (Langer and Vacanti 1993; Hubbell 1995). These chemicals affect the stem cells and control the differentiation process. It is proven in in vitro and in vivo studies that calcium ceramics, which are one of the said chemicals, are very effective for bone mineralization (Jarcho 1981). Chemicals and materials, which are used for bone mineralization in some studies, can also be effective in tooth mineralization (Holtgrave and Donath 1995). Main Differentiation Chemicals are Dexamethasone, Beta Glycerophosphate, and Ascorbic Acid. Differentiation of the stem cells into hard tissues and mineralization thereof are enabled by Dexamethasone, Ascorbic acid and Beta glycerophosphate (Park 2012). NaF, Pluronic F68, Melatonin, Calcium Hydroxide are chemicals which enhance activity. NaF enables hardening and strength of the tooth cells (Salmela, Lukinmaa et al. 2011). Pluronic F68, enhances permeability and flexibility of the cell membrane and thus enhances effect of the active chemicals entering into the cell (Dogan, Yalvac et al. 2012; Kabanov and Alakhov 2002). Melatonin is effective in cell differentiation and protection of cell viability (Kumasaka, Shimozuma et al. 2010). Calcium hydroxide is the major chemical necessary for mineral formation (Peng, Liu et al. 2011).

The International patent document no. WO2011062147 discloses a method for inducing differentiation of dental pulp cells into odontoblasts and regeneration of dentin.

The International patent document no. WO2012045097 discloses a method of forming a mineralized material in osteoblasts and odontoblasts.

### Summary of the Invention

An objective of the present invention is to provide a dental differentiation product by which tooth cell can be obtained effectively with the adult stem cells used in tissue engineering.

Another objective of the present invention is to provide a dental differentiation product which increases odontogenic potential and mineralization of the cells in cell therapy studies.

A further objective of the invention is to provide a dental differentiation product which can be used within the materials for coating the tooth root or crown in endodontics.

### Detailed Description of the Invention

"Dental Differentiation Product" developed to fulfill the objectives of the present invention is illustrated in the accompanying figures, in which:
**Figure 1** is the view of toxicity of the dental differentiation product on the cells when the chemicals therein are applied alone and in combination (Mel: Melatonin, Ca(OH)2: Calcium hydroxide, NaF: Sodium fluoride, DFS: Dental differentiation product).
**Figure 2** is the view of the effect of the dental differentiation product on the alkaline phosphatase activity (DFS: dental differentiation product).
**Figure 3** is the view of the effect of the dental differentiation product on the mineralization of cells (DFS: dental differentiation product).
**Figure 4** is the view of the effect of the dental differentiation product on the mRNA levels expressed by the cells (DFS: dental differentiation product,

COL1A: Collagen Type 1, DSPP: Dentin Sialophospho Protein, BMP2: Odontogenic Differentiation Protein 2, ALP: Alkaline Phosphatase).

### Experimental Study

In tissue engineering, both hard (mineral) and soft (pulp) tissues of the tooth should be formed for a functional tooth. By means of this invention, tooth cells can be obtained effectively with adult stem cells used in tissue engineering.

### Cell Culture

Cells which were isolated and characterized in advance (Tasl₁ et al, 2012) were cultured at 37°C and 5% CO₂ in Dulbecco modified essential medium (DMEM) (Invitrogen, Carlsbad, CA) including 10% fetal bovine serum (FBS), 2 mM of 1-glutamine (Invitrogen) and 1% penicillin, streptomycin and amphotericin (PSA) (Invitrogen, Carlsbad, CA).

### Isolation and Characterization of hTGSC

hTGSC was isolated from the third molar tooth of a 13 year old male subject obtained after the patient and his family signed the consent form received from Istanbul University (Istanbul, Turkey) Ethics Committee. Isolation and characterization of hTGSC was performed as previously stated in the literature (Tasl₁ et al, 2012). The stem cells were grown in Dulbecco's modified essential medium (DMEM) which contained 10% fetal bovine serum and 1% PSA (penicillin, streptomycin and amphotericin), in an incubator at a temperature of 37°C and in 5% CO₂ atmosphere. After the cells were trypsinized, they were treated for 1 hour with primer antibodies diluted in PBS (PBS; cat #10010, pH 7,4; Invitrogen). CD29 (cat #BD556049), CD34 (cat #SC-51540), CD45 (cat #SC-70686), CD90 (cat #SC-53456), CD105 (cat #SC-71043), CD133 (cat #SC-65278), CD166 (cat #SC-53551) (Santa Cruz Biotechnology Inc, Santa Cruz, CA), and CD73 (cat #D 550256) (Zymed, San Francisco, CA) primary antibodies were used for characterization. The cells were washed with PBS three times for removing the excess unbonded primary antibodies in the medium. Then the cells were treated at 4°C for 1 hour with secondary antibodies conjugated with fluorescein isothiocyanate (cat. #SC-2989, 200µg/0.5mL; Santa Cruz Biotechnology). Only CD29 was not treated with a secondary antibody because it was in the form of a monoclonal antibody conjugated with phycoerythrin comprising chromophore. Flow cytometry analysis was performed by using Becton Dickinson FACSCalibur (Becton Dickinson, San Jose, CA) system.

### Preparation of the Product

The product is comprised of a liquid mixture comprising C₁₃H₁₆N₂O₂ (melatonin), Ca(OH)₂ (calcium hydroxide), C₂₂H₂₉FO₅ (dexamethasone), C₃H₇Na₂O₆P ·5H₂O (disodium beta-glycerophosphate hydrate), NaF (sodium fluoride) and Pluronic chemicals. These chemicals were weighed separately and dissolved in the medium. 50nM C₁₃H₁₆N₂O₂ was dissolved in 100% ethanol, 1,8mM Ca(OH)₂ was dissolved in water. All the other chemicals were mixed in 10nM C₂₂H₂₉FO₅, 5mM C₃H₇Na₂O₆P ·5H₂O, 80nM NaF and 10% Pluronic medium at room temperature. The resulting solution can be used by preparing into a 5 times larger density and diluting every time in the medium.

### Determining Cell Toxicity

The effect of the prepared product on cell viability was determined by using the MTS (material testing systems) method given in the literature (Yalvac et al., 2009). The molecules used in the product were prepared alone or in combination in the medium and applied on hTGSCs (human tooth germ stem cells) which were seeded on 96-well culture plates by counting at a concentration of 4000 cells per each well. The response of the cells to toxicity of the molecules was determined by measuring cell viability for 3 days. Cell viability was determined by using a method called MTS which measures mitochondrial dehydrogenase enzyme activity of the cell. The MTS substance added onto the cells together with the medium results in colored formazan crystals formation as an indicator of cell viability. The resulting color change was evaluated based on the absorbance measurement by using ELISA plate reader. The obtained results were analyzed.

### Dental Differentiation

For differentiation, the stem cells were seeded in a 6-well culture plate at a concentration of 50000 cells per each well. The special dental differentiation mixture was prepared in DMEM containing 5mM C₃H₇Na₂O₆P·5H₂O (Sigma, USA), 50ug/ml C₆H₈O₆ (Sigma, USA),10nM C₂₂H₂₉FO₅ (Sigma, USA),1,8mM Ca(OH)2, 80nM NaF, 100mg/ml Pluronic F68 and conventionally used differentiation solution was prepared in DMEM containing 5mM C₃H₇Na₂O₆P·5H₂O (Sigma, USA), 50ug/ml C₆H₈O₆ (Sigma, USA) and 10nM C₂₂H₂₉FO₅ (Sigma, USA). The seeded cells were added to the cells every other day for a period 14 days.

### Measurement of Alkaline Phosphatase Activity

After being treated with the differentiation product for a period of 14 days, the cells were mixed with Triton-X-100 prepared in 0.2% PBS for 30 minutes at room temperature. 20 µl of the formed cell lysate was placed into 96 wells and 80 µl ALP determining product was added (BioAssay Systems, USA). After allowing it to stand for 15 minutes in the dark, measurement was conducted at 405 nm wavelength.

### Von Kossa Straining

After being treated with the differentiation product for a period of 14 days, the cells were washed with PBS and were incubated for 30 minutes with 2% paraformaldehyde (Sigma, US) and fixed. After the fixing process was finished, 250µl 3% silver nitrate product was added to the cells that were washed with PBS and the cells were subjected to UV light for 1 hour. 250µl sodium was added to the cells, which were washed with PBS, for 2 minutes. Then 250µl nuclear fast red stain was added and allowed to stand for 5 minutes at room temperature. After washing with PBS, the black and brown minerals were observed with light microscope.

### Real Time Polymerase Chain Reaction

When stems cells are differentiating, they lose their own properties and acquire new properties. These properties are both at morphological level and gene expression level. Type 1 collagen (de Medeiros, Nonaka et al.), dentin sialophosphoprotein (Zhu, Nie et al. 2008) and bone morphogenic protein 2 genes are among the most important reagents used for verifying dental differentiation. They are expressed by the mineralized cell which is one of the first stages of dental differentiation. Type 1 collagen is a protein which takes part in formation of mineral and dentin and enables formation of the collagen structure (Linde and Goldberg 1993; Butler 1995; Butler 1998). DSPP which is synthesized by dental stem cells and released throughout dental differentiation is a protein which takes part in mineral formation (Veis 1993; Butler 1995; Butler 1998). DSPP is the most important factor in mineralization of dentin (Butler 1989; Veis 1993; Butler 1995; Butler 1998). BMP 2 affects dental differentiation and formation of dentin-like structures (Gronthos, Arthur et al.; Wang, Liu et al.). Real time PCR analyses were performed according to the literature by using SYBR Green method (Yalvac et al, 2010). The primers belonging to the collagen type 1, dentinsialophospho protein, bone morphogenic protein 2 and alkaline phosphate genes were designed using Primer BLAST software (The National Center for Biotechnology= NCBI). Total RNAs were isolated from the cells on which gel combination was applied and cDNA was synthesized. The synthesized cDNAs were mixed with primers in Fermentas Maxima SYBR Green mixture product such that the final volume will be 20µl and the expression levels of the genes were analyzed by using BIO-RAD device.

### Experiment results

### Cell Viability Test

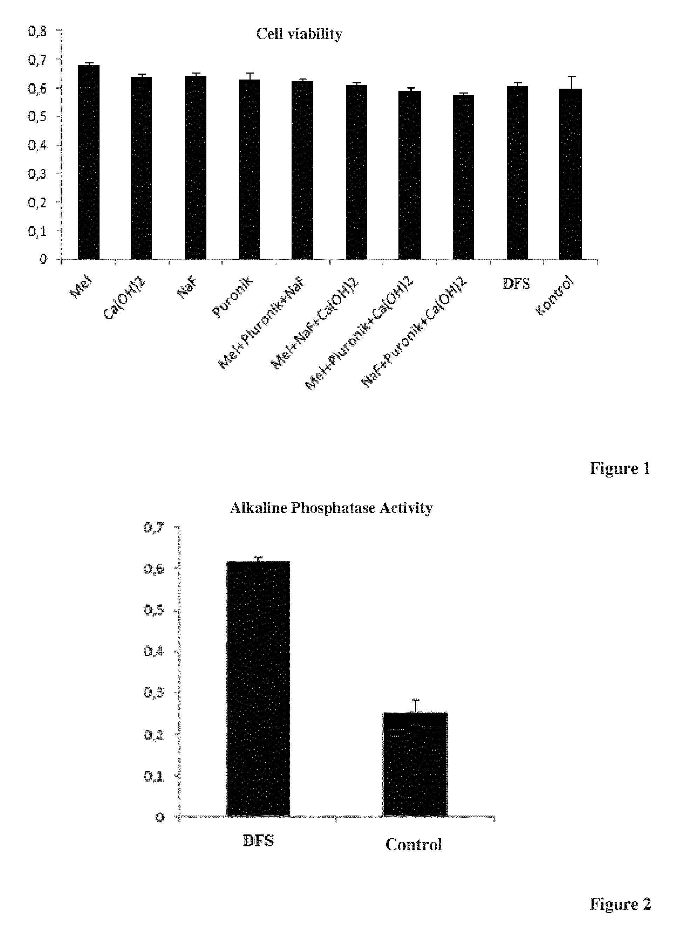

Cell viability test shows that no toxicity was observed at the end of 3 days when the chemicals were applied alone or in combinations (Figure 1).

### ALP test

The group treated with the dental differentiation mixture newly prepared at the end of 14 days in measurement of Alkaline phosphatase is named as DFS, while the group treated with the conventional dental differentiation product is named PC. As can be seen from the obtained results, the present product increased ALP activity by 2 and a half times via differentiation (Figure 2).

### Von Kossa Staining

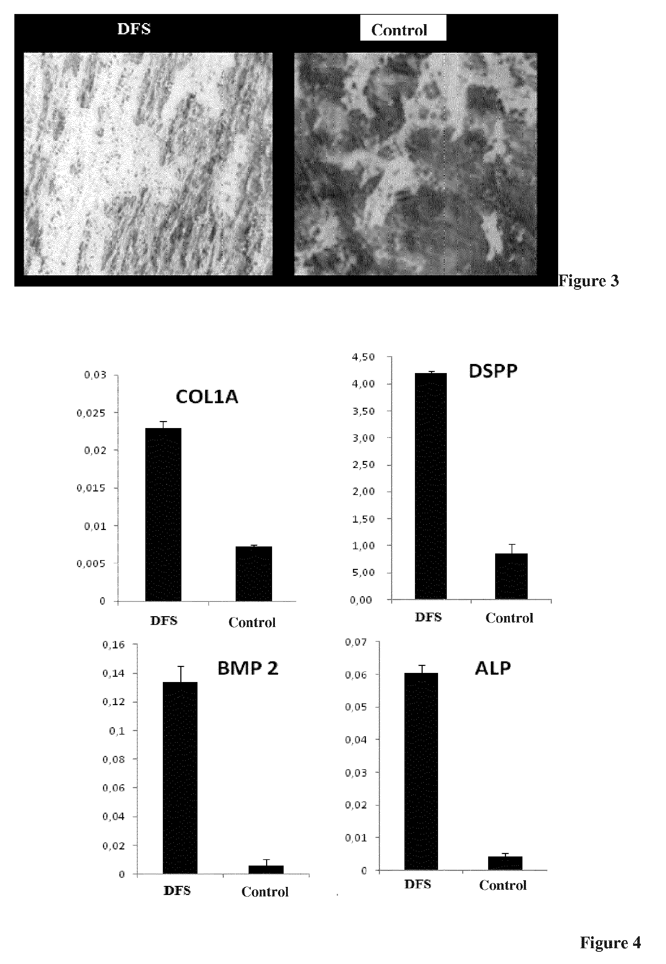

Mineralization was observed in both of the differentiation groups at the end of 14 days. As it is seen in the figure, the amount of mineral formed by DFS is more (Figure 3).

### PCR results

Upon examining differentiation at mRNA level, the new product increased COL1A expression level by 3.5 times, DSPP expression level by 4.5 times, BMP 2 expression level by 11 times and ALP expression level by 12 times (Figure 4).

### Application of the Invention

The product of the invention can be used in differentiation of stem cells in cell culture in in vitro conditions. It can also be used in in vitro and in vivo tissue engineering studies.

The product of the present invention can be a toothpaste, mouthwash, dental filling materials, root canal therapy materials, tooth strengthening creams or gels.

Dental differentiation product can be in the form of a solution as well as in the form of a concentrate or lyophilized powder. The lyophilized powder form extends shelf life of the product. Powder form may be purposeful in cases where use of intense amount is required.

### References

About, I., M. J. Bottero, et al. (2000). "Human dentin production in vitro." Exp Cell Res 258(1): 33-41.
About, I. and T. A. Mitsiadis (2001). "Molecular aspects of tooth pathogenesis and repair: in vivo and in vitro models." Adv Dent Res 15: 59-62.
Ahmed, F., P. Alexandridis, et al. (2001). "The ability of poloxamers to inhibit platelet aggregation depends on their physicochemical properties." Thromb Haemost 86(6): 1532-1539.
Alhadlaq, A. and J. J. Mao (2003). "Tissue-engineered neogenesis of human-shaped mandibular condyle from rat mesenchymal stem cells." J Dent Res 82(12): 951-956.
Alliot-Licht, B., G. Bluteau, et al. (2005). "Dexamethasone stimulates differentiation of odontoblast-like cells in human dental pulp cultures." Cell Tissue Res 321(3): 391-400.
Bartholomew, A., C. Sturgeon, et al. (2002). "Mesenchymal stem cells suppress lymphocyte proliferation in vitro and prolong skin graft survival in vivo." Exp Hematol 30(1): 42-48.
Batrakova, E. V., S. Li, et al. (1999). "Pluronic P85 increases permeability of a broad spectrum of drugs in polarized BBMEC and Caco-2 cell monolayers." Pharm Res 16(9): 1366-1372.
Bocelli-Tyndall, C., A. Barbero, et al. (2006). "Human articular chondrocytes suppress in vitro proliferation of anti-CD3 activated peripheral blood mononuclear cells." J Cell Physiol 209(3): 732-734.
Braccini, A., D. Wendt, et al. (2005). "Three-dimensional perfusion culture of human bone marrow cells and generation of osteoinductive grafts." Stem Cells 23(8): 1066-1072.
Butler, W. T. (1989). "The nature and significance of osteopontin." Connect Tissue Res 23(2-3): 123-136.
Butler, W. T. (1995). "Dentin matrix proteins and dentinogenesis." Connect Tissue Res 33(1-3): 59-65.
Butler, W. T. (1998). "Dentin matrix proteins." Eur J Oral Sci 106 Suppl 1: 204-210.
Chattopadhyay, D., J. F. Rathman, et al. (1995). "The protective effect of specific medium additives with respect to bubble rupture." Biotechnol Bioeng 45(6): 473-480.
Cihova, M., V. Altanerova, et al. "Stem cell based cancer gene therapy." Mol Pharm 8(5): 1480-1487.
De Bari, C., F. Dell'Accio, et al. (2001). "Multipotent mesenchymal stem cells from adult human synovial membrane." Arthritis Rheum 44(8): 1928-1942.
de Medeiros, A. M., C. F. Nonaka, et al. "Expression of extracellular matrix proteins in ameloblastomas and adenomatoid odontogenic tumors." Eur Arch Otorhinolaryngol 267(2): 303-310.
Djouad, F., C. Bouffi, et al. (2009). "Mesenchymal stem cells: innovative therapeutic tools for rheumatic diseases." Nat Rev Rheumatol 5(7): 392-399.
Foster, B., T. Cosgrove, et al. (2009). "Pluronic triblock copolymer systems and their interactions with Ibuprofen." Langmuir 25(12): 6760-6766.
Friedenstein, A. J., R. K. Chailakhjan, et al. (1970). "The development of fibroblast colonies in monolayer cultures of guinea-pig bone marrow and spleen cells." Cell Tissue Kinet 3(4): 393-403.
Ghebeh, H., J. Gillis, et al. (2002). "Measurement of hydrophobic interactions of mammalian cells grown in culture." J Biotechnol 95(1): 39-48.
Gigout, A., M. D. Buschmann, et al. (2008). "The fate of Pluronic F-68 in chondrocytes and CHO cells." Biotechnol Bioeng 100(5): 975-987.
Glennie, S., I. Soeiro, et al. (2005). "Bone marrow mesenchymal stem cells induce division arrest anergy of activated T cells." Blood 105(7): 2821-2827.
Graziano, A., R. d'Aquino, et al. (2008). "Dental pulp stem cells: a promising tool for bone regeneration." Stem Cell Rev 4(1): 21-26.
Gronthos, S., A. Arthur, et al. "A method to isolate and culture expand human dental pulp stem cells." Methods Mol Biol 698: 107-121.
Gronthos, S., J. Brahim, et al. (2002). "Stem cell properties of human dental pulp stem cells." J Dent Res 81(8): 531-535.
Gronthos, S., M. Mankani, et al. (2000). "Postnatal human dental pulp stem cells (DPSCs) in vitro and in vivo." Proc Natl Acad Sci U S A 97(25): 13625-13630.
Ha, J. C., S. Y. Kim, et al. (1999). "Poly(ethylene oxide)-poly(propylene oxide)-poly(ethylene oxide) (Pluronic)/poly(epsilon-caprolactone) (PCL) amphiphilic block copolymeric nanospheres. I. Preparation and characterization." J Control Release 62(3): 381-392.
Hargreaves, K. M., T. Geisler, et al. (2008). "Regeneration potential of the young permanent tooth: what does the future hold?" Pediatr Dent 30(3): 253-260.
Hokett, S. D., M. F. Cuenin, et al. (2000). "Pluronic polyol effects on human gingival fibroblast attachment and growth." J Periodontol 71(5): 803-809.
Holtgrave, E. A. and K. Donath (1995). "Response of odontoblast-like cells to hydroxyapatite ceramic granules." Biomaterials 16(2): 155-159.
Hristov, M., W. Erl, et al. (2003). "Endothelial progenitor cells: isolation and characterization." Trends Cardiovasc Med 13(5): 201-206.
Hubbell, J. A. (1995). "Biomaterials in tissue engineering." Biotechnology (N Y) 13(6): 565-576.
Huss, R. (2000). "Perspectives on the morphology and biology of CD34-negative stem cells." J Hematother Stem Cell Res 9(6): 783-793.
Husseini, G. A., G. D. Myrup, et al. (2000). "Factors affecting acoustically triggered release of drugs from polymeric micelles." J Control Release 69(1): 43-52.
Ikeda, E., K. Yagi, et al. (2008). "Multipotent cells from the human third molar: feasibility of cell-based therapy for liver disease." Differentiation 76(5): 495-505.
Jarcho, M. (1981). "Calcium phosphate ceramics as hard tissue prosthetics." Clin Orthop Relat Res(157): 259-278.
Jiang, M., L. Lv, et al. "Induction of pluripotent stem cells transplantation therapy for ischemic stroke." Mol Cell Biochem 354(1-2): 67-75.
Jones, E. A., A. English, et al. (2004). "Enumeration and phenotypic characterization of synovial fluid multipotential mesenchymal progenitor cells in inflammatory and degenerative arthritis." Arthritis Rheum 50(3): 817-827.
Jones, E. A., S. E. Kinsey, et al. (2002). "Isolation and characterization of bone marrow multipotential mesenchymal progenitor cells." Arthritis Rheum 46(12): 3349-3360.
Jordan, M., H. M. Eppenberger, et al. (1994). "Interactions between animal cells and gas bubbles: The influence of serum and pluronic F68 on the physical properties of the bubble surface." Biotechnol Bioeng 43(6): 446-454.
Kabanov, A. V. and V. Y. Alakhov (2002). "Pluronic block copolymers in drug delivery: from micellar nanocontainers to biological response modifiers." Crit Rev Ther Drug Carrier Syst 19(1): 1-72.
Kataoka, K., T. Matsumoto, et al. (2000). "Doxorubicin-loaded poly(ethylene glycol)-poly(beta-benzyl-L-aspartate) copolymer micelles: their pharmaceutical characteristics and biological significance." J Control Release 64(1-3): 143-153.
Kinnaird, T., E. Stabile, et al. (2004). "Marrow-derived stromal cells express genes encoding a broad spectrum of arteriogenic cytokines and promote in vitro and in vivo arteriogenesis through paracrine mechanisms." Circ Res 94(5): 678-685.
Krampera, M., S. Glennie, et al. (2003). "Bone marrow mesenchymal stem cells inhibit the response of naive and memory antigen-specific T cells to their cognate peptide." Blood 101(9): 3722-3729.
Kwon, G. S., M. Naito, et al. (1995). "Physical entrapment of adriamycin in AB block copolymer micelles." Pharm Res 12(2): 192-195.
Laino, G., R. d'Aquino, et al. (2005). "A new population of human adult dental pulp stem cells: a useful source of living autologous fibrous bone tissue (LAB)." J Bone Miner Res 20(8): 1394-1402.
Laino, G., A. Graziano, et al. (2006). "An approachable human adult stem cell source for hard-tissue engineering." J Cell Physiol 206(3): 693-701.
Langer, R. and J. P. Vacanti (1993). "Tissue engineering." Science 260(5110): 920-926.
Le Blanc, K., I. Rasmusson, et al. (2004). "Treatment of severe acute graft-versus-host disease with third party haploidentical mesenchymal stem cells." Lancet 363(9419): 1439-1441.
Li, X., Y. Huang, et al. (2009). "Self-assembly and characterization of Pluronic P105 micelles for liver-targeted delivery of silybin." J Drug Target 17(10): 739-750.
Linde, A. and M. Goldberg (1993). "Dentinogenesis." Crit Rev Oral Biol Med 4(5): 679-728.
Lovschall, H., T. A. Mitsiadis, et al. (2007). "Coexpression of Notch3 and Rgs5 in the pericyte-vascular smooth muscle cell axis in response to pulp injury." Int J Dev Biol 51(8): 715-721.
Maccario, R., M. Podesta, et al. (2005). "Interaction of human mesenchymal stem cells with cells involved in alloantigen-specific immune response favors the differentiation of CD4+ T-cell subsets expressing a regulatory/suppressive phenotype." Haematologica 90(4): 516-525.
Maeda, H., L. W. Seymour, et al. (1992). "Conjugates of anticancer agents and polymers: advantages of macromolecular therapeutics in vivo." Bioconjug Chem 3(5): 351-362.
Maitra, B., E. Szekely, et al. (2004). "Human mesenchymal stem cells support unrelated donor hematopoietic stem cells and suppress T-cell activation." Bone Marrow Transplant 33(6): 597-604.
Marin, A., M. Muniruzzaman, et al. (2001). "Acoustic activation of drug delivery from polymeric micelles: effect of pulsed ultrasound." J Control Release 71(3): 239-249.
Marin, A., M. Muniruzzaman, et al. (2001). "Mechanism of the ultrasonic activation of micellar drug delivery." J Control Release 75(1-2): 69-81.
Meier, S. J., T. A. Hatton, et al. (1999). "Cell death from bursting bubbles: role of cell attachment to rising bubbles in sparged reactors." Biotechnol Bioeng 62(4): 468-478.
Michaels, J. D., J. E. Nowak, et al. (1995). "Analysis of cell-to-bubble attachment in sparged bioreactors in the presence of cell-protecting additives." Biotechnol Bioeng 47(4): 407-419.
Michaels, J. D., J. F. Petersen, et al. (1991). "Protection mechanisms of freely suspended animal cells (CRL 8018) from fluid-mechanical injury. Viscometric and bioreactor studies using serum, pluronic F68 and polyethylene glycol." Biotechnol Bioeng 38(2): 169-180.
Miller, D. W., E. V. Batrakova, et al. (1999). "Inhibition of multidrug resistance-associated protein (MRP) functional activity with pluronic block copolymers." Pharm Res 16(3): 396-401.
Mitsiadis, T. A. and C. Rahiotis (2004). "Parallels between tooth development and repair: conserved molecular mechanisms following carious and dental injury." J Dent Res 83(12): 896-902.
Miura, M., S. Gronthos, et al. (2003). "SHED: stem cells from human exfoliated deciduous teeth." Proc Natl Acad Sci U S A 100(10): 5807-5812.
Mizrahi, A. (1975). "Pluronic polyols in human lymphocyte cell line cultures." J Clin Microbiol 2(1): 11-13.
Munshi, N., N. Rapoport, et al. (1997). "Ultrasonic activated drug delivery from Pluronic P-105 micelles." Cancer Lett 118(1): 13-19.
Murhammer, D. W. and C. F. Goochee (1990). "Structural features of nonionic polyglycol polymer molecules responsible for the protective effect in sparged animal cell bioreactors." Biotechnol Prog 6(2): 142-148.
Nosrat, I. V., C. A. Smith, et al. (2004). "Dental pulp cells provide neurotrophic support for dopaminergic neurons and differentiate into neurons in vitro; implications for tissue engineering and repair in the nervous system." Eur J Neurosci 19(9): 2388-2398.
Nosrat, I. V., J. Widenfalk, et al. (2001). "Dental pulp cells produce neurotrophic factors, interact with trigeminal neurons in vitro, and rescue motoneurons after spinal cord injury." Dev Biol 238(1): 120-132.
Papaccio, G., A. Graziano, et al. (2006). "Long-term cryopreservation of dental pulp stem cells (SBP-DPSCs) and their differentiated osteoblasts: a cell source for tissue repair." J Cell Physiol 208(2): 319-325.
Peretti, K. L., H. Ajiro, et al. (2005). "A highly active, isospecific cobalt catalyst for propylene oxide polymerization." J Am Chem Soc 127(33): 11566-11567.
Perry, B. C., D. Zhou, et al. (2008). "Collection, cryopreservation, and characterization of human dental pulp-derived mesenchymal stem cells for banking and clinical use." Tissue Eng Part C Methods 14(2): 149-156.
Pittenger, M. F., A. M. Mackay, et al. (1999). "Multilineage potential of adult human mesenchymal stem cells." Science 284(5411): 143-147.
Ramirez, O. T. and R. Mutharasan (1990). "The role of the plasma membrane fluidity on the shear sensitivity of hybridomas grown under hydrodynamic stress." Biotechnol Bioeng 36(9): 911-920.
Rapoport, N., A. Marin, et al. (2002). "Intracellular uptake and trafficking of Pluronic micelles in drug-sensitive and MDR cells: effect on the intracellular drug localization." J Pharm Sci 91(1): 157-170.
Rapoport, N., A. P. Marin, et al. (2000). "Effect of a polymeric surfactant on electron transport in HL-60 cells." Arch Biochem Biophys 384(1): 100-108.
Rapoport, N. and L. Pitina (1998). "Intracellular distribution and intracellular dynamics of a spin-labeled analogue of doxorubicin fluorescence and EPR spectroscopy." J Pharm Sci 87(3): 321-325.
Rapoport, N. Y., J. N. Herron, et al. (1999). "Micellar delivery of doxorubicin and its paramagnetic analog, ruboxyl, to HL-60 cells: effect of micelle structure and ultrasound on the intracellular drug uptake." J Control Release 58(2): 153-162.
Reynolds, T. (1995). "Polymers help guide cancer drugs to tumor targets--and keep them there." J Natl Cancer Inst 87(21): 1582-1584.
Roche, S., M. Provansal, et al. (2006). "Proteomics of primary mesenchymal stem cells." Regen Med 1(4): 511-517.
Scherlund, M., A. Brodin, et al. (2000). "Micellization and gelation in block copolymer systems containing local anesthetics." Int J Pharm 211(1-2): 37-49.
Seshi, B., S. Kumar, et al. (2000). "Human bone marrow stromal cell: coexpression of markers specific for multiple mesenchymal cell lineages." Blood Cells Mol Dis 26(3): 234-246.
Sezgin, Z., N. Yuksel, et al. (2006). "Preparation and characterization of polymeric micelles for solubilization of poorly soluble anticancer drugs." Eur J Pharm Biopharm 64(3): 261-268.
Shi, S. and S. Gronthos (2003). "Perivascular niche of postnatal mesenchymal stem cells in human bone marrow and dental pulp." J Bone Miner Res 18(4): 696-704.
Smith, A. J., N. Cassidy, et al. (1995). "Reactionary dentinogenesis." Int J Dev Biol 39(1): 273-280.
Smith, A. J. and H. Lesot (2001). "Induction and regulation of crown dentinogenesis: embryonic events as a template for dental tissue repair?" Crit Rev Oral Biol Med 12(5): 425-437.
Tasl1 PN, S Tapsm, S Demirel, ME Yalvaç, S Akyuz, A Yarat ve F Sahin. (2012). Isolation and Characterization of Dental Pulp Stem Cells from a Patient with Papillon-Lefèvre Syndrome. J Endod 39:31-38.
Tecles, O., P. Laurent, et al. (2005). "Activation of human dental pulp progenitor/stem cells in response to odontoblast injury." Arch Oral Biol 50(2): 103-108.
Thesleff, I. and P. Sharpe (1997). "Signalling networks regulating dental development." Mech Dev 67(2): 111-123.
Thomson, B. M., J. Bennett, et al. (1993). "Preliminary characterization of porcine bone marrow stromal cells: skeletogenic potential, colony-forming activity, and response to dexamethasone, transforming growth factor beta, and basic fibroblast growth factor." J Bone Miner Res 8(10): 1173-1183.
Thomson, J. A., J. Itskovitz-Eldor, et al. (1998). "Embryonic stem cell lines derived from human blastocysts." Science 282(5391): 1145-1147.
Tziafas, D., A. Alvanou, et al. (1995). "Induction of odontoblast-like cell differentiation in dog dental pulps after in vivo implantation of dentine matrix components." Arch Oral Biol 40(10): 883-893.
Tziafas, D., A. J. Smith, et al. (2000). "Designing new treatment strategies in vital pulp therapy." J Dent 28(2): 77-92.
Veis, A. (1993). "Mineral-matrix interactions in bone and dentin." J Bone Miner Res 8 Suppl 2: S493-497.
Wang, J., X. Liu, et al. "The odontogenic differentiation of human dental pulp stem cells on nanofibrous poly(L-lactic acid) scaffolds in vitro and in vivo." Acta Biomater 6(10): 3856-3863.
Wang, W., G. J. Tudryn, et al. "Thermally driven ionic aggregation in poly(ethylene oxide)-based sulfonate ionomers." J Am Chem Soc 133(28): 10826-10831.
Wei, G. and P. X. Ma (2008). "Nanostructured Biomaterials for Regeneration." Adv Funct Mater 18(22): 3566-3582.
Woods, E. J., B. C. Perry, et al. (2009). "Optimized cryopreservation method for human dental pulp-derived stem cells and their tissues of origin for banking and clinical use." Cryobiology 59(2): 150-157.
Yalvac, M. E., A. A. Rizvanov, et al. (2009). "Potential role of dental stem cells in the cellular therapy of cerebral ischemia." Curr Pharm Des 15(33): 3908-3916.
Yamaza, T., A. Kentaro, et al. "Immunomodulatory properties of stem cells from human exfoliated deciduous teeth." Stem Cell Res Ther 1(1): 5.
Yang, X., X. F. Walboomers, et al. (2008). "Non-viral bone morphogenetic protein 2 transfection of rat dental pulp stem cells using calcium phosphate nanoparticles as carriers." Tissue Eng Part A 14(1): 71-81.
Zappia, E., S. Casazza, et al. (2005). "Mesenchymal stem cells ameliorate experimental autoimmune encephalomyelitis inducing T-cell anergy." Blood 106(5): 1755-1761.
Zhang, W., X. F. Walboomers, et al. (2008). "Hard tissue formation in a porous HA/TCP ceramic scaffold loaded with stromal cells derived from dental pulp and bone marrow." Tissue Eng Part A 14(2): 285-294.
Zhu, F., R. R. Nie, et al. (2008). "[Expression of odontogenic and osteogenic genes in bone marrow mesenchymal stem cells with overexpression of mouse dentin sialophosphoprotein]." Sichuan Da Xue Xue Bao Yi Xue Ban 39(2): 290-293.
Zuk, P. A., M. Zhu, et al. (2002). "Human adipose tissue is a source of multipotent stem cells." Mol Biol Cell 13(12): 4279-4295.

## Claims

1. Dental differentiation product which obtains tooth cell from adult stem cells and is comprised of a mixture of C₁₃H₁₆N₂O₂ (melatonin), Ca(OH)₂ (calcium hydroxide), C₂₂H₂₉FO₅ (dexamethasone), C₃H₇Na₂O₆P ·5H₂O (disodium beta-glycerophosphate hydrate), NaF (sodium fluoride) and pluronic.

2. Dental differentiation product according to Claim 1 which can be used in differentiation of stem cells in cell culture in in vitro conditions.

3. Dental differentiation product according to Claim 1 to 2, which can be applied in in vitro and in vivo tissue engineering studies.

4. Dental differentiation product according to Claim 1 to 3, which can be in the form of a solution, concentrate or lyophilized powder.

5. Dental differentiation product according to Claim 1 to 4, which can be a toothpaste, mouthwash, dental filling materials, root canal therapy materials, tooth strengthening creams or gels.
